# EUROPEAN PATENT APPLICATION

(11) **EP 2 690 065 A1**
(43) Date of publication of application: **29.01.2014**
(21) Application number: 12177440.0
(22) Date of filing: 23.07.2012
(51) Int. Cl.: C01B 33/141, C12N 11/14

(54) **Silica-based gel with entrapped sensitive material and method for its production**

(71) Applicant: Fachhochschule Bielefeld, 33615 Bielefeld (DE)
(72) Inventor: Patel, Anant, 33604 Bielefeld (DE); Müller, Christiane, 33613 Bielefeld (DE); Kroke, Edwin Prof. Dr., 09633 Halsbrücke (DE)
(74) Representative: Kröncke, Rolf

(57) **Abstract**

The present invention relates in a first aspect to a silica-based gel with entrapped sensitive material. Said sensitive material is in particular a light-, pH-, and/or temperature sensitive material. Said silica-based gel with entrapped sensitive material is obtained by using a Si-N precursor having at least two amino groups whereby gelling is conducted in the presence of said sensitive material. In another aspect, the present invention relates to a method for preparing said silica-based gel with entrapped sensitive material. Finally, the present invention provides specific uses of the silica-based gel with entrapped sensitive material according to the present invention.

## Description

The present invention relates in a first aspect to a silica-based gel with entrapped sensitive material. Said sensitive material is in particular a light-, pH-, and/or temperature sensitive material. Said silica-based gel with entrapped sensitive material is obtained by using a Si-N precursor having at least two amino groups whereby gelling is conducted in the presence of said sensitive material. In another aspect, the present invention relates to a method for preparing said silica-based gel with entrapped sensitive material. Finally, the present invention provides specific uses of the silica-based gel with entrapped sensitive material according to the present invention.

### Prior art

Entrapping sensitive material, e.g. immobilization of biomolecules or immobilization of other light-, pH- and/or temperature sensitive material is a demanding task in the art. That is, immobilization of biomolecules within polymer matrices has gained considerable importance in various fields of biotechnical processes. For example photosynthetically active components which can absorb light and transfer energy, may be immobilized (entrapped) within suitable immobilization compounds, like polymer matrices to allow the provision of suitable systems, like immobilized photosynthetically active components within a matrix, for biotechnical processes.

For example, in the field of energy transfer processes, it is desired to provide systems containing photosynthetically active components. Said photosynthetically active components can absorb light and transfer energy accordingly. All eukaryotic cells using photosynthesis contain photosynthetically active components, like the light harvesting complexes to optimize light utilization. The light harvesting complexes are protein pigment complexes containing different pigments like chlorophyll A/B and carotenoids. However, photosynthetically active components are extremely unstable and need to be stabilised outside of the cell, in order to use these highly effective components e.g. in third generation photovoltaics and artificial photosynthesis, respectively.

The immobilized sensitive material includes active components, like the mentioned photosynthetically active components, but also optically and electrochemically active material.

Entrapment or encapsulation of sensitive material in polymers, like biopolymers, is known in the art. Beside encapsulation of whole cells, cell compartments or cell organelles as well as biological macromolecules including proteins, enzymes, etc. or other sensitive materials have been entrapped or encapsulated. Numerous biopolymers as well as synthetic polymers are described in the art useful for encapsulation of functional components, in particular, living cells or bioactive materials. In addition, systems have been described wherein the sensitive materials have been encapsulated in biopolymers first and said encapsulated sensitive materials are coated with e.g. silica allowing to associate soft biocompatible components, like alginate, with a tough thermostable non-swelling component like silica.

However, sol-gel entrapment or encapsulation of sensitive material is still a challenging matter. A comparison of different processes for sol-gel encapsulation of bacteria is provided e.g. in Coiffier et. al. J. Mater Chem. 2001,11,2039 - 2044. As identified therein, the amount of methanol produced by hydrolysis and condensation of TMOS (the Si-O precursor used therein) could exceed the tolerable dose, thus, being detrimental for the functional components entrapped in the polymer system.

Further, Coradin et. al., Current Nanoscience, 2006, 2, 219 ― 230 discuss various problems when preparing sol-gel biopolymer silica composites. It is identified that obstacles in the preparation processes are the presence of alcohol in the various reaction media, unfavourable pH, temperature etc. As identified, approaches were proposed including thin film deposition, evaporation of alcohol before biological addition, synthesis of new precursor bearing biocompatible alcohol substituents as well as substitution of silicon alkoxide by aqueous precursors, like silicates or colloidal silica. However, the outcome of said various approaches are still not favourable. That is, the use of TMOS or TEOS as typical examples of Si-O precursor materials requires specific conditions for the hydrolysis and condensation. Firstly, temperatures of above 100°C are necessary to allow condensation. Secondly, the reaction requires solubilisation in solvents, typically organic solvents such as ethanol and/or ethers. Besides, a pH value in the acid range is typically applied, in particular, at pH 2 or below in order to allow sufficient reaction times and to avoid any gelling of the mixture resulting in an extended reaction time. That is, low or high pH ranges are necessary to catalyse the reaction with high reactivity of the reactant. In addition, to avoid undesired gelling responsible for prolonged reaction time, organic solvents, like alcohols such as ethanol or methanol, ethers such as THF or other organic solvents beside water or other aqueous solvents are required for solubilisation of TMOS and other precursor molecules based on Si-O precursors only.

Recently, Meunier et. al. Chem. Commun., 2010, 46, 3843 - 3859, discuss the use of living hybrid materials capable of energy conversion and CO₂ assimilation. This paper reviews work on the fabrication of photobiochemical hybrid materials via immobilization of photosynthetically active entities within silica materials and summarizing the viability and productivity of these active entities post encapsulation and evaluating their efficiency as a principal component of a photobioreactor. As identified therein, there is still a problem on controlling the formation of gels from silica precursors and silica nanoparticles for entrapping the living or functional components. It is identified therein further that there is still an ongoing need for providing polymer systems, either as coated polymer systems or non-coated systems having improved activity while showing enhanced lifetime.

Also it is discussed that the results described therein represent a significant advance towards the final goal, long lasting semi-artificial photobioreactors that can advantageously exploit solar radiation to convert polluting carbondioxide into useful bio fuels, sugars or biochemical metabolites, the system provided therein based on TMOS precursors is still insufficient on maintaining activity over the time.

The present invention is directed to new silica-based gel with entrapped sensitive material whereby said sensitive material is in particular light-, pH- and/or temperature- sensitive.

In another aspect, the present invention is directed to methods for preparing said silica-based gel with entrapped sensitive material whereby the methods allow the preservation of the activity of said entrapped sensitive material.

### Brief summary of the present invention

In a first aspect, a silica-based gel with entrapped sensitive material is provided. Said sensitive material is in particular a light-, pH- and/or temperature sensitive material. The silica-based gel with entrapped sensitive material is characterized in that said gel is a silica-based gel obtained by using Si-N precursors having at least two amino groups whereby gelling is conducted in the presence of said light-, pH- and/or temperatures sensitive material allowing entrapment of said material in the silica-based gel.

It has been recognized by the present inventors that by using a Si-N precursor material, it is possible to overcome the problem associated with the sole use of Si-O precursor material, like TMOS, in the art. In particular, due to the fact that in contrast to the use of TMOS reduced or, preferably, no toxic compounds like aliphatic alcohol molecules are produced and released, thus, the sensitive material present in the silica-based gel remain functional and are not detoriated or destroyed due to said toxic compounds or low pH values or high temperature necessary for gelling and polymerisation, i.e. due to the lesser reactivity of the Si-O precursors at neutral pH and low temperature.

Hence, the detrimental side effects of using only Si-O material are avoided. In particular, bio compatibility is enhanced and, it is possible to provide photosensitive material like photosynthetically active material in a silica-based gel accordingly.

It is particularly preferred, that the entrapped sensitive material is a biological material, like a photosynthetically active material. Moreover, it is preferred that the Si-N precursor is an amino silan of the general formula (I) as described below. In particular, it is preferred that the gel is a transparent gel, like a transparent xerogel.

In another aspect, the present invention provides a method for the preparation of an entrapped sensitive material as defined herein in a silica-based gel. Said method comprises a step of forming
a) a liquid sol from at least the Si-N precursor and a solvent, like an aqueous solvent, in particular, water;
b) adding and mixing the sensitive material, in particular, the light-, pH- and/or temperature-sensitive material into said sol;
c) gelling of said mixture of sol and the sensitive material, in particular, the light-, pH- and/or temperature-sensitive material;
d) obtaining silica-based gel with entrapped sensitive material.

Finally, the present invention relates to the use of said silica-gel with entrapped sensitive material in biosensors, medical, pharmaceutical, agricultural, food and cosmetic applications, photonic devices, optical information processing and optical materials as well as in bioreactors.

### Brief description of the drawings

Figure 1: Figure 1 shows the influence of Si-N precursor concentration on the gel and glass transparency as described in example 2.
Figure 2: Figure 2 shows the UV-Vis spectra of free and immobilized chlorophyllin as described in example 4.
Figure 3: Figure 3 shows the UV-Vis spectra of free and immobilized chlorophyllin and light-harvesting complex as described in example 4.
Figure 4: Figure 4 shows the absorption of free and immobilized chlorophyllin (630 nm) and light-harvesting complex (671 nm) as a function of time according to example 5.
Figure 5: Figure 5 shows the yield over the time. The yield is an indicator for the photosynthetic activity of the cells as described in example 6.
Figure 6: Figures 6a and 6b are the solid state nuclear magnetic resonance spectra of the glassy structure according to the present invention, i.e. ²⁹Si NMR and ¹³C NMR solid state spectra, respectively.

### Detailed description of the present invention

In a first aspect, a silica-based gel with entrapped sensitive material is provided. Said gel is a silica-based gel obtained by using a Si-N precursor having at least two amino groups. The gelling of the silica-based gel is conducted in the presence of the sensitive material.

The presence of the Si-N precursor material having at least two amino groups allows providing an environment maintaining the functional activity of the sensitive material entrapped therein.

As used herein, the term "entrapped" is synonymously used with the term "immobilized" whereby the sensitive material is covered fully by the silica-based gel.

It is preferred, that the sensitive material is a light-, pH- and/or temperature-sensitive material. In this connection, the term light-, pH- and/or temperature-sensitive material refers to a material having a functionality which is sensitive to light, pH and/or temperature. That is, the functionality refers to a specific feature of the material, e.g. its activity, colour, physical or chemical or biological properties which are altered e.g. at low or high pH, high temperature, for example above 50°C, like above 40°C as well as being sensitive to high concentrations of harmful or toxic by-products of Si-O polymerisation. Thus, the desired properties (specific characteristic) of said sensitive material may be impaired or even destroyed at conditions required for hydrolysis and condensation of sole Si-O precursors.

In particular, the sensitive material according to the present invention is a photosensitive material. In another aspect, the sensitive material is a biological material. In this connection, the term "biological material" refers to materials derived from or being of biological origin or having biological or biochemical functionality, inter alia to biologicals including catalytic antibodies, DNA, RNA, antigens, life bacterial, fungal, plant and animal cells as well as whole protozoa for various uses in red, whiteand green biotechnology, catalytically active components, like catalysts or enzymes useful in all types of reactions, in particular, to chlorophylline, light-harvesting complex, phycocyanine, enzyme, nucleic acids, cells and cell organelles, like *Chlamydomonas reinhardtii.* Furthermore, biological materials include plants or parts of plants.

In a particular preferred embodiment the sensitive material according to present invention is a photosynthetically active material. For example, the photosynthetically active material is a chlorophylline or a light-harvesting complex as well as cells or cell organelles of plant or microorganism origin, like *Chlamydomonas reinhardtii.* The photosynthetically active material according to the present invention may be present in form of the isolated molecules or may be present in form of whole cells containing said photosynthetically active material.

It is preferred that the remaining functionality after gelling is of at least 50%, like at least 60%, e.g. at least 70%, like at least 80%, in particular, of at least 90%, like 95% compared to the functionality before gelling according to the present invention.

In particular, the sensitive material is at least a part of a photosynthetically active component which can absorb light and transfer energy useful in third generation photovoltaics and for artificial photosynthesis as well as in other biomimetic applications. For example the photosynthetically active material may be components of the whole photosystems of eukaryotic cells. The present inventors recognized, that it is possible to obtain improved silica-based gels entrapping (encapsulating) sensitive material, e.g. in form of particles, layers or films by polymerisation and/or solvolysis-condensation reactions of Si-N precursors having at least two amino groups. In this connection it is noted that the term "entrapped" and "encapsulated" are used herein interchangeably unless otherwise identified.

The Si-N precursors are favourable in view of the production process, like the hydrolysis and condensation of the Si-N precursor and, in particular, the Si-N precursors are favourable in view of the sensitivity of the sensitive material, in particular, in view of the sensitivity against light-, pH- and/or temperature. In particular, the Si-N precursors are favourable in view of sensitivity of the biological material and/or photosensitive material to low pH values, acids as well as high temperature and compounds being harmful or toxic to the material, like the lower alcohols methanol or ethanol formed during the hydrolysis of TMOS, TEOS or related (functionalised) alkoxy silanes.

In one aspect of the present invention, the silica-based gel is formed of Si-N precursor exclusively. However, another embodiment of the present invention is directed to a silica-based gel obtained by gelling of Si-N precursor in combination with other suitable precursor material including Si-O precursor. However, it is preferred that the amount of Si-N precursor is above 25%, preferably at least 50 %, preferably above 70 %, like more than 80 %, e.g. more than 90 % of the total amount of precursors. For example, the ratio of Si-N precursor and other precursors including Si-O precursor is selected in a way that the gelling is conducted at temperatures not being detrimental to the sensitive material, e.g. at temperatures below 50°C like below 40°C, e.g. at room temperature. Moreover, the ratio of Si-N precursor and Si-O precursor or other precursor material is selected in a way that the pH is in the range of from 6 to 8, preferably, at a pH of around 7.

The present inventors recognised that substituting at least a part of known Si-O precursor, preferable all of the Si-O precursor, with Si-N precursor having at least two amino groups result in a silica-based gel entrapping sensitive material wherein the environmental and reaction conditions are less detrimental to the sensitive material than using Si-O precursor molecules alone.

As used herein, the term "polymerisation" refers to a reaction of forming a polymer including the process of hydrolysis and condensation as well as solvolysis-condensation.

As used herein, the term "Si-O precursor" refers to silane derivatives containing at least two, like three or four, reactive ―OR residues with R being H or any other substituent, like hydrocarbon, as a leaving group during polymerisation.

In addition, the term "Si-N precursor" refers to silane derivatives having at least two amino groups whereby said amino groups may be primary, secondary or tertiary amino groups. The Si-N precursor useful according to the present invention has at least two amino groups allowing condensation of said precursor molecules to silica polymers whereby primary amines, secondary amines or ammonia are generated.

In this connection, the term "sensitive material" refers to material which looses function upon exposure to environmental conditions, like pH, light, or temperature. The term "biological material" refers inter alia to sensitive material comprising whole cells and cell compartments, proteins, in particular, enzymes, nucleic acids, secondary metabolites. In particular, the sensitive material is a material, e.g. molecule, which is sensitive to low pH values and/or high temperature, e.g. above 50°C, like above 40°C as well as being sensitive to harmful or toxic by-products like methanol or ethanol. That is, the functionality, e.g. the activity or specific characteristic, of said sensitive material is pH-sensitive and/or temperature-sensitive as well as being sensitive to toxic or harmful compounds. Thus, the desired properties (specific characteristic) of said sensitive material may be impaired or even destroyed at pH or temperature conditions required for hydrolysis and condensation of Si-O precursor molecules. In a preferred embodiment, the sensitive material are biological materials, in particular, cells, cellular components, and/or biologically active molecules, like, enzymes or molecules involved in photosynthesis.

As used herein, the term "comprising" or "comprise" or "containing" or "contain" include the embodiment of "consisting" or "consist".

The present inventors identified that using the Si-N precursor molecules instead of Si-O precursor molecules allow to obtain silica-based gels entrapping sensitive materials under reaction conditions more favourable for said sensitive materials present in the silica-based gel according to the present invention. In contrast to the required reaction conditions when using alkoxide components, i.e. reaction conditions at or below pH 2 and temperatures above 100°C, the hydrolysis and condensation of the Si-N precursor molecules, like aminosilane molecules, can be performed quickly at room temperature and can be started at neutral pH values, thus, allowing to retain the activity of sensitive material. In addition, the toxicity of by-products of hydrolysis and condensation of the Si-O precursor, e.g. methanol or ethanol, is avoided. In contrast, the side products of the hydrolysis and condensation of the Si-N precursor, like secondary amines, primary amines or ammonia are less harmful or toxic to the sensitive material of the silica-based gel according to the present invention.

The gel according to the present invention is preferably a gel wherein the content of nitrogen in the silica-based gel is at least 5%, like at least 10% higher than the content of nitrogen in silica-based gel based on a Si-O precursor. That is, it is preferred that the content of nitrogen in the silica-gel with entrapped sensitive material according to the present invention is at least 5%, like at least 10% higher than the content of nitrogen in a similar system wherein said gel is obtained by condensation of Si-O precursor. Preferably, the content of nitrogen is at least 15% higher, like at least 20% higher, in particular, at least 25% higher. Alternatively or in addition, the amount of nitrogen in the silica-based gel in the absence of a sensitive material is at least 0.1 wt.-%, like at least 0.2, 0.3 or at least 0.5 wt.-% of the weight of the total silica-based gel not containing sensitive material. It is preferred that the amount of nitrogen is at least 1.0 wt.-% of the weight of the total coated polymer system, like 1.5 wt.-% or at least 2.0 wt.-%.

The Si-N precursors are preferably aminosilane components or oligo- and polysilazane components or mixtures thereof.

That is, the Si-N precursor is preferably an aminosilane component of the general formula (I)

(I) AₓSi(NR₁R₂)₄₋ₓ

wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, A may be an aromatic residue, preferably A is independently selected from optionally substituted C₁-C₂₀-alkyl-, C₆-C₁₂-aryl, in particular preferred a methyl, ethyl, propyl, butyl or phenyl residue. Substituents include hydrocarbons, like branched or linear alkyl, alkenyl alkoxy, hydroxyl, halogens, but also amine, amid, imid groups as well isocyanate groups, etc. Preferably, the hydrocarbon is a hydrocarbon of C₁ to C₆ including C₂, C₃, C₄ and C₅.
x is an integer of 0, 1 or 2

R₁ and R₂ are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R₁ and R₂ may be an aromatic residue, preferably R₁ and R₂ is independently selected from hydrogen, optionally substituted C1-C20-alkyl-, C6-C12-aryl, in particular preferred a hydrogen, methyl, ethyl, propyl, butyl, hexyl or phenyl residue, whereby the substituents are preferably the substituents as defined above namely hydrocarbons, like branched or linear alkyl, alkenyl alkoxy, hydroxyl, halogens, but also amine, amid, imid or ether groups as well isocyanate groups, etc. further including functionalized primary amines.

In particular, it is preferred that A is methyl, ethyl or propyl and/or R1 and R2 are independently from each other selected from hydrogen, n-propyl, isopropyl and hexyl whereby at least one of R1 and R2 are not hydrogen.

It is preferred that x is an integer of 0 or 1, thus the aminosilane is a triaminosilane or tetraaminosilane, i.e. A-Si(NR₁R₂)₃ or Si(NR₁R₂)₄.

In another aspect, the Si-N precursor is an oligo- and/or polysilazane. For example, the oligo- or polysilazane is an perhydrated oligo- or polysilazane based on (SiH₂-NH₂)ₙ chains or rings. Oligo- und polysilazanes are inorganic polymers consisting of Si-N-Si-chains. Unsubstituted, i.e. perhydrated oligo- und polysilazanes based on (SiH₂-NH₂)ₙ chains and rings are very reactive and, thus, are preferred according to the present invention.

For example, perhydropolysilazane (PHPS) as given below are used:

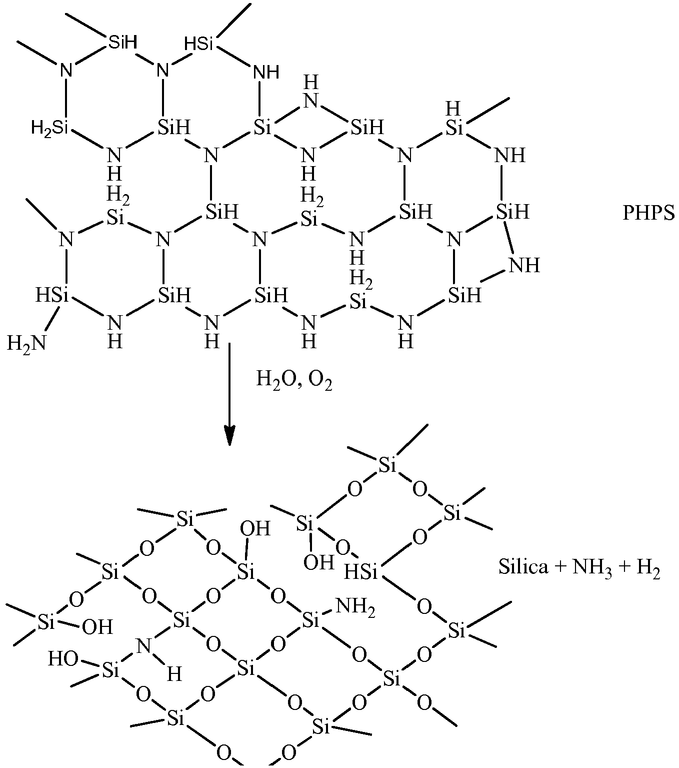

It is preferred that polysilazanes of the general formula (II) are used.

-(SiR¹R²-NR³)ₙ- (II)

wherein R¹, R², R³ are identical or different from each other and are independently from each other selected from hydrogen, unsubstituted or substituted hydrocarbons, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R¹ and R² may be an aromatic residue, preferably R¹ and R² is independently selected from optionally substituted C₁-C₂₀-alkyl-, C₆-C₂₄-aryl, in particular preferred a methyl, ethyl, propyl, butyl, hexyl or phenyl residue, whereby the substituent may be any one as defined above for R (R1, ...). In addition, R¹, R², R³ may represent an aprotic group, like halogens, ―CN, NO₂, OR, COOR, heterocyclic groups, or alkyl, alkenyl, or alkinyl groups, whereby R is a linear or branched, saturated or unsaturated hydrocarbon, like C₁-C₆ alkyl, C₁ to C₂₀ alkyl or alkenyl.

Preferred components of the aminosilane group are ―NHR with R = alkyl, especially R = methyl, ethyl, n-propyl, n-butyl, iso-butyl, t-butyl, n-pentyl, n-hexyl, cyclohexyl.

Further, the Si precursor molecules may comprise bridging components like Si-B-Si moieties whereby B represents suitable bridging elements corresponding to substituent A as defined above. Further, halogenated silanes as well known to the skilled person may be used as precursor molecules, like chlorosilanes.

The Si-N precursor molecule release primary amine, secondary amines or ammonia depending on the residues present at the N-atom of the amino group, which are less detrimental to the functional components than the side products released when using Si-O precursor molecules, like methanol or ethanol. Moreover, due to higher reactivity of the aminosilanes, the gelling and/or coating and/or immobilisation process is much faster under the same pH- and temperature conditions.

The primary or secondary amine hydrocarbon components or ammonia released as side products of hydrolysis and condensation of Si-N precursor compounds are less reactive and less harmful or toxic to the functional component. In addition, hydrolysis and condensation of the Si-N precursor is possible at lower temperature and at pH values of neutral pH. Moreover, the coating processes are fast and efficient, thus, preserving functional activity of the functional components present in the system according to the present invention. Furthermore, if mixtures of Si-N precursor and chlorosilanes are used, the formed hydrochloric acid HCl will react with the amine or ammonia leading to ammonium compounds and thus keeping the pH in the neutral range not causing an acidification which would harm the sensitive biological material.

In a preferred embodiment, the sensitive material in a silica-based gel is a gel wherein the gel is a water containing gel or said gel is a xerogel. As used herein, the term "xerogel" refers to a solid form derived from a gel by drying. Sometimes the xerogels also referred to as glass. That is, the silica-based gel may be in form of a hydrogel or aquagel formed by using water or aqueous solutions as a solvent (and reactant). In addition, the gel may be in form of a organogel, that is, the solvent is an organic solvent, like an aprotic solvent, e.g. hexane, glycerine or propandiol as well as mixtures of suitable solvents.

As used herein, the term "optionally substituted" refers to embodiments wherein the residue referred to can be substituted with a hydrocarbon substituent, like branched or linear alkyl, alkenyl, alkoxy, hydroxyl, halogen, amine, amid, imid or isocyanate group. Preferably the substituent is any one of a C1 to C6 hydrocarbon, including C2, C3, C4, C5.

It is preferred, that the gel is formed from a liquid sol containing at least the Si-N precursor and an aqueous solvent, in particular, water.

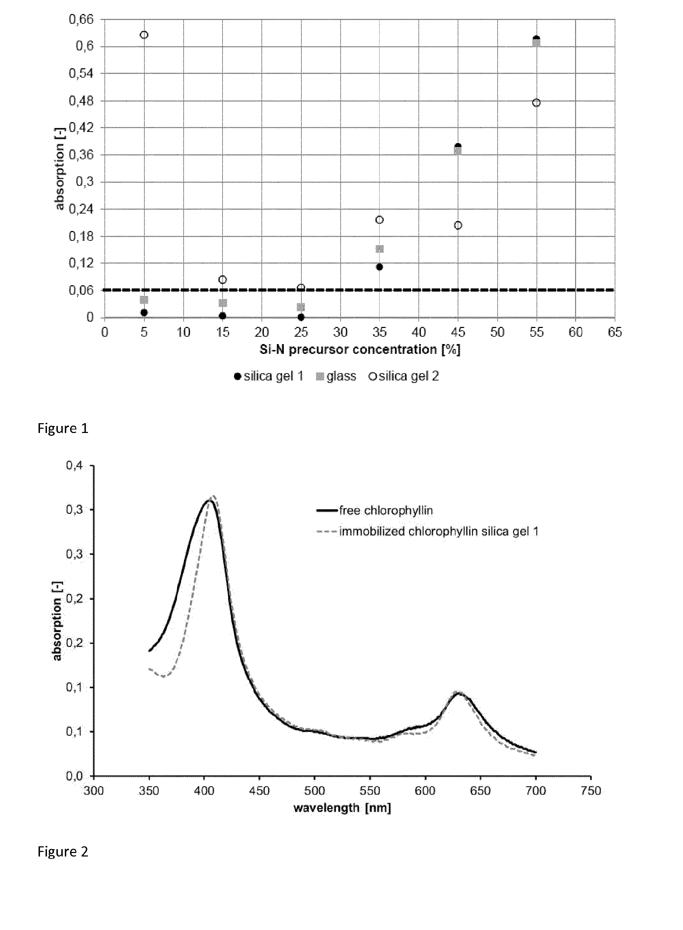

It is particularly preferred that the gel is formed by a Si-N precursor only in an aqueous solvent, in particular, water. In a particular preferred embodiment, the silica-based gel is obtained by forming a liquid sol from the Si-N precursor only in water. It is particular preferred, that the amount of precursor, in particular, the Si-N precursor is of from 5 to 25 weight-% in said aqueous solvent. It has been determined that the concentration of the precursor may influence the transparency of the formed gel. That is, at concentration of from 5 to 55 wt-% of the overall amount of precursor, a gel is formed while transparency decreases above 25 wt-% precursor. In this connection, it is referred to figure 1 demonstrating the same.

In a preferred embodiment, the silica-based gel with entrapped sensitive material is a transparent gel, like a transparent xerogel.

In another aspect, the present invention relates to a method for the preparation of a silica-based gel with entrapped sensitive material. In particular, said sensitive material is a light-, pH- and/or temperatures-sensitive material. The method according to the present invention for the preparation of said silica-based gel containing sensitive material comprises a step of forming a liquid sol from at least the Si-N precursor and the solvent. It is preferred, that the solvent is an aqueous solvent, in particular, water. Moreover it is preferred that the Si-N precursor does not contain other precursor materials, however, it is possible that less than 75 % Si-O precursor or other types of precursor, e.g. 50% or less, like 30 % or less, preferably 20 % or less may be present as well.

After mixing and obtaining a liquid sol the sensitive material is added and mixed said sol. Thereafter, gelling of said mixture of sol and the sensitive material is conducted for obtaining silica-based gel with entrapped sensitive material.

Optionally, the preparation may contain additionally the step of filtration of the liquid sol after addition and mixing of the sensitive material. In addition, it is preferred that formation of the liquid sol is conducted whereby the container containing the liquid sol is covered by a cover.

After adding and mixing the sensitive material, the sol containing the sensitive material is allow to gel. It is preferred, that gelling is promoted or expedited by drying, for example convection drying. Drying is conducted preferably at temperatures below 50°C, preferably below 40°C, e.g. at room temperature or up to 35°C, for example, not more than 30°C.

Gelling of the sol containing the sensitive material, preferably, under convection drying, allows to obtain a silica-based gel with entrapped sensitive material. The gel may be in form of a hydrogel or, after further drying, may be in form of a xerogel, e.g. being in a glassy shape having an amorphous chemical structure.

When forming the liquid sol from at least the Si-N precursor and an organic solvent using the steps outlined above, a gel is obtained while using an aqueous solvent, a gel is obtained with, after further drying, allows to obtain a glassy gel or xerogel.

In a preferred embodiment, gelling is effected at a pH of 2 to 12 preferably 4 to 10, like at a pH of from 6 to 8 and/or at a temperature of -20 to 80°C, in particular, 0°C to 60°C, preferably 20°C to 40°C.

It is particularly preferred, that the Si-N precursor used in the method according to the present invention is an aminosilane of the general formula (I)

AₓSi(NR₁R₂)₄₋ₓ (I)

wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue furthermore,

A may be an aromatic residue, preferably A is independently selected from optionally substituted C₁-C₂₀-alkyl-, C₆-C₂₄-aryl, in particular preferred a methyl, ethyl, propyl, vinyl, butyl or phenyl residue,
x is an integer of 0, 1 or 2
R₁ and R₂ are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R₁ and R₂ may be an aromatic residue, preferably R₁ and R₂ is independently selected from optionally substituted C₁-C₂₀-alkyl-, C₆-C₂₄-aryl, in particular preferred a methyl, ethyl, propyl, butyl, hexyl or phenyl residue, it is particular preferred that the Si-N precursor having at least two amino groups as an oligo- or polysilazane.

The silica-based gel with entrapped sensitive material according to the present invention is particularly useful in biosensors, photonic devices, optical information processing and optical materials as well as in bioreactors and in photovoltaics. In particular, the gel according to present invention represents effective components in third generation photovoltaics and in artificial photosynthesis. It is preferred, that the sensitive materials are components of the photosynthesis system including chlorophyllin, light harvesting complexes as well as phycocyanin.

The gel containing the sensitive material according to the present invention allows to provide a system having a spatial distribution of the sensitive material, like the components of photosynthesis and elements of photovoltaics as desired. Moreover, as demonstrated herein, the entrapped sensitive material allows to demonstrate higher stability while not changing of the desired properties has been shown.

Not to be bound by theory, it is demonstrated in the example that the immobilization does not influence the absorption spectra of e.g. the light harvesting complex when entrapped in a silica-based gel using Si-N precursor molecules. Moreover, it is demonstrated herein, that stabilization of the entrapped material is possible. One of the major problems in the process of third generation photovoltaics and artificial photosynthesis is the instability of the photosynthetic active components necessary for absorption of light and energy transfer. With the novel silica-based gel disclosed herein it is possible to stabilize said photosynthetically active components while not changing the absorption maxima after gel formation. In contrast to gels based on Si-O precursors only, the detrimental effects of the pH value present during hydrolysis and formation of the gel as well as the toxic side products, like the formation of lower alcohols, can be avoided.

The invention will be described further by way of example without limiting the present invention thereon.

### Examples

### Example 1 Formation of silica-based gel and xerogel

The transparent silica-based gels and glass were prepared with a novel sol-gel method. The silica-based gel 1 and glass were obtained as follows: 2,5 g of Si-N precursor was mixed with 7,5 g of demineralized water and stirred 18 hours for obtaining a transparent liquid sol 1 having a pH above 7. 2 g of the resulting transparent liquid sol 1 is weighed in a glass petri dish and dried by convection drying (room temperature) to promote the gelation and glass formation. The silica-based gel 2 was formed in combination with a 10 % (w/w) acid catalyzed tetraethylorthosilicate sol 2. 2,5 g of Si-N precursor was mixed with 7,5 g demineralized water and was mixed with 10 g of a 10 % (w/w) sol 2 and stirred 18 hours. 5 g of the resulting sol 3 was mixed with 22 mL hydrochloric acid (c=0,01 mol/L) and 5 mL sulfuric acid (c=0,05 mol/L). This mixture was acidified with concentrated sulfuric acid and then the pH value dropped with sol 3 exactly to pH 7. 2 g of the resulting transparent liquid sol 4 is weighed in a glass petri dish and dried by convection drying (room temperature) to promote the gelation.

The following compositions have been used above:
**Sol 1:** 2,5 g of Si-N precursor and 7,5 g of demineralized water,
**Sol 2:** 10 % acid catalyzed tetraethylorthosilicate
**Sol 3:** 10 g of a 25 % (w/w) sol 1 was mixed with 10 g of a 10 % (w/w) sol 2
**Sol 4:** 5 g of the resulting sol 3 was mixed with 22 mL hydrochloric acid (c=0,01 mol/L) and 5 mL sulfuric acid (c=0,05 mol/L). This mixture was acidified with concentrated sulfuric acid and then the pH value dropped with sol 3 exactly to pH 7
**Silica-based gel 1:** 2,5 g of Si-N precursor and 7,5 g of demineralized water, a homogenous solid gel
**Silica-based gel 2:** 5 g of the resulting sol 3, 22 mL hydrochloric acid (c=0,01 mol/L), 5 mL sulfuric acid (c=0,05 mol/L), the pH value exactly to pH 7, a homogenous solid gel
**Silica-based glass 1:** 2,5 g of Si-N precursor and 7,5 g of demineralized water, a homogenous solid xerogel

### Example 2 Transparency of silica gels and xerogel

The influence of Si-N precursor concentration on the gel and glass transparency was studied using UV-Vis spectroscopy at a specific wavelength of 860 nm (Figure 1). The determined absorption is a measure of the transparency of a silica gel and glass. The limit lies at an absorption of 0,06, below this value a silica gel and glass is considered transparent.

These results show that silica-based gel 1 and glass with a concentration of from 5 % to 25 % is transparent and above 35 % the transparency decreases. The silica-based gel 2 is less transparent like the silica-based gel 1, and the optimal transparency is at a concentration of 25 % Si-N precursor.

### Example 3 Characterization of silica gel and xerogel

| | **gelling time ± standard deviation [h]** | **water conent ± standard deviation [%]** | **dynamic viscosity at a shear rate of 0,1 s⁻¹ [Pa s]** | **bulk density [g/cm³]** |
|---|---|---|---|---|
| **Silica-based gel 1** | 1,27 ± 0,10 | 75,68 ± 3,62 | 2070 x10⁵ | 1,15 |
| **Silica-based gel 2** | 0,17 ± 0,03 | 95,70 ± 0,97 | 832,86 | 0,95 |
| **Silicab-based glass 1** | 1,68 ± 0,10 | 28,49 ± 6,35 | not determined | |

The gelling time was determined by weighing 2 g of the resulting transparent liquid sol 1 or sol 4 in a glass petri dish and drying by convection drying (room temperature). The water content was determined by the residual moisture analyzer. The dynamic viscosity was determined by a rotational rheometer. The bulk density was calculated by determining the mass of the gel using an analytical balance and then, the dimension of the gel was measured with calipers.

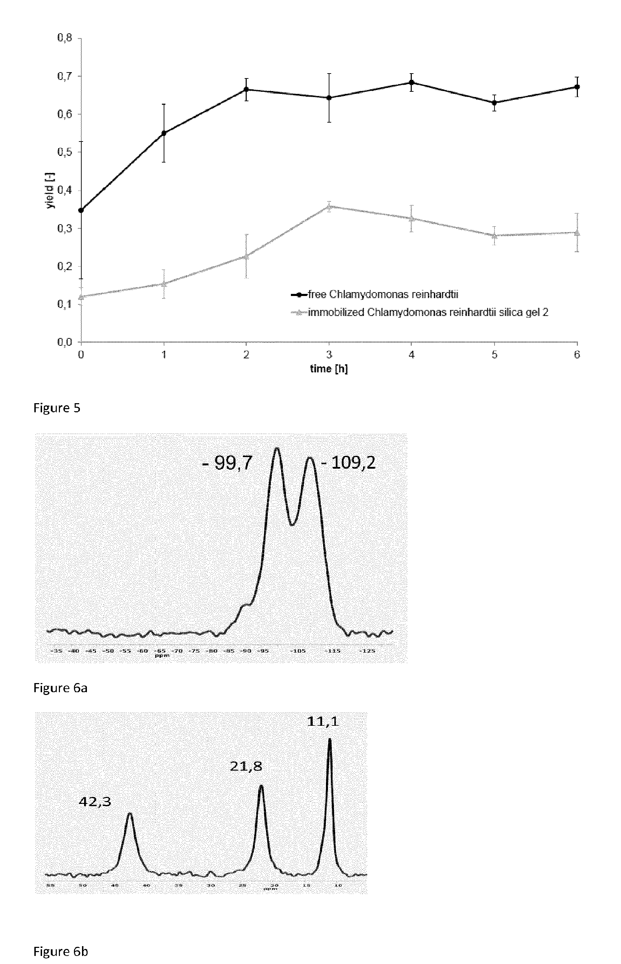

The xerogel 1 analysis by ²⁹Si solid state nuclear magnetic resonance spectroscopy indicates that this xerogel structure is a three-dimensional silica based network, see figure 6a. Figure 6b (¹³C solid state nuclear magnetic resonance spectroscopy) shows that the network contains propyl group, which suggesting that the network was prepared from the Si-N precursor.

### Example 4 Immobilization of chlorophyllin and a light-harvesting complex (Lhc) in silica-based gels

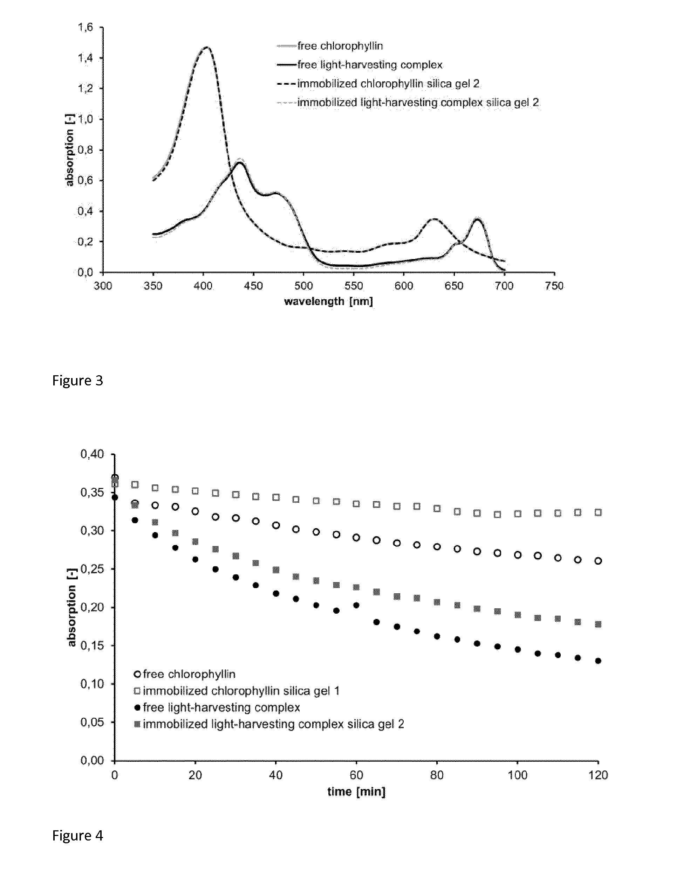

The influence of immobilization on the absorption spectra of chlorophyllin and Lhc was studied using the UV-Vis spectroscopy. Chlorophyllin (Sigma-Aldrich) was entrapped in silica-based gel 1 and 2 and the Lhc in silica-based gel 2, see Figure 2, 3.

Free Lhc has typically absorbance maxima at 430 nm-470 nm (carotenoids) and 650 nm-671 nm (chlorophyll a/b), corresponding to the different pigments bound to the complex. Free chlorophyllin has typically absorbance maxima at 405 nm and 630 nm.

Immobilized chlorophyllin and Lhc showed no change of absorbance maxima after the gel formation. This indicates that the pigment and the protein-pigment structure remain unchanged in the gel directly after immobilization.

### Example 5 Stabilization of chlorophyllin and a light-harvesting complex (Lhc) in silica-based gels

The influence of light exposure on the stability of photosynthetically active components (PACs) was studied using UV-Vis spectroscopy at specific wavelengths (chlorophyllin: 630 nm, Lhc: 671 nm).

Light absorption of free chlorophyllin and free Lhc was reduced by 29 % and 62 % after 2 h, respectively. After entrapment of chlorophyllin, the light absorption was reduced by 10 % after 2 h. Entrapment of Lhc resulted in absorption reduced by 51 % after 2 h, Figure 3. These results indicate a stabilizing effect of the novel silica-based gels.

### Example 6 Immobilization of Chlamydomonas reinhardtii

Chlamydomonas reinhardtii were obtained as a cell suspension having a concentration of 1x10⁷ cells/ml. The cell suspension was centrifuged and mixed with 10 mL of the sol 4. The cells were entrapped and gelled as described above.

First, the photosynthetic activity (measured with the Photosynthesis Yield Analyzer) of the cells after the immobilization was investigated. It has been found that initially, the photosynthetic yield was reduced after immobilization (from 0.35 to 0.12) but recovered subsequently to a certain extent (Yield 0.12 enhanced to 0.3), indicating a regeneration of the photosynthetic activity of the cells.

Furthermore, the photosynthetic activity of the entrapped cells was measured by polarographic oxygen measurements. These results show a clear detection of respiratory consumption (-1.5 nmol O₂/mL/min) which suggests a general vitality of the immobilized cells. These preliminary data indicate that this hydrogen-producing strain of *C. reinhardtii* can be immobilized within a novel silica-based gel.

## Claims

1. Silica-based gel with entrapped sensitive material, in particular, light-, pH-, and/or temperature-sensitive material wherein said gel is a silica-based gel obtained by using a Si-N precursor having at least two amino groups and gelling of the precursor is conducted in the presence of said light-, pH- and/or temperature-sensitive material.

2. The silica-based gel with entrapped sensitive material, in particular, light-, pH-, and/or temperature-sensitive material in a silica-based gel according to claim 1 wherein said light-, pH- and/or temperature-sensitive material is a photosensitive material.

3. The silica-based gel with entrapped sensitive material, in particular, light-, pH-, and/or temperature-sensitive material, according to claim 1 or 2 wherein said light-, pH- and/or temperature-sensitive material is a biological material.

4. The silica-based gel with entrapped sensitive material, in particular, light-, pH-, and/or temperature-sensitive material, according to any one of the preceding claims wherein said light-, pH- and/or temperature-sensitive material is a photosynthetically active material.

5. The silica-based gel, in particular with entrapped sensitive material, in particular, light-, pH-, and/or temperature-sensitive material, according to any one of the preceding claims wherein the gel is an aqueous solvent and/or organic solvent containing gel or said gel is a xerogel.

6. The silica-based gel with entrapped sensitive material, in particular, light-, pH-, and/or temperature-sensitive material, according to any one of the preceding claims wherein the Si-N precursor is an aminosilane of the general formula (I)
AₓSi(N R¹R²)₄₋ₓ (i)
wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, A may be an aromatic residue, preferably A is independently selected from optionally substituted C₁-C₂₀-alkyl-, C₆-C₂₄-aryl, in particular preferred a methyl, ethyl, propyl, vinyl, allyl, butyl or phenyl residue,
X is an integer of 0, 1 or 2
R¹ and R² are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R¹ and R² may be an aromatic residue, preferably R₁ and R₂ is independently selected from optionally substituted C₁-C₂₀-alkyl-, C₆-C₂₄-aryl, in particular preferred a methyl, ethyl, propyl, butyl, hexyl or phenyl residue.

7. The silica-based gel with entrapped sensitive material, in particular, light-, pH-, and/or temperature-sensitive material, according to claim 1, wherein the Si-N precursor having at least two amino groups is an oligo- or polysilazane.

8. The silica-based gel with entrapped sensitive material, in particular, light-, pH-, and/or temperature-sensitive material, according to any one of the preceding claims wherein the gel is a transparent gel, in particular, a transparent xerogel.

9. A method for the preparation of a silica-based gel with entrapped sensitive material, in particular, light-, pH-, and/or temperature-sensitive material, comprising the steps of
a) forming a liquid sol from at least the Si-N precursor and a solvent, like an aqueous solvent, in particular, water;
b) adding and mixing the sensitive material, in particular, light-, pH-, and/or temperature-sensitive material into said sol;
c) gelling of said mixture of sol and the sensitive material, in particular, light-, pH-, and/or temperature-sensitive material;
d) obtaining silica-based gel with entrapped sensitive material, in particular, light-, pH-, and/or temperature-sensitive material.

10. The method according to claim 9 wherein gelling is effected at a pH of 2 to 12, preferably 4 to 10, like at a pH of from 6 to 8 and/or at a temperature of -20°C to 80°C, in particular, 0°C to 60°C, preferably 20°C to 40°C.

11. The method according to any one of claims 9 or 10, **characterized in that** the Si-N precursor is an aminosilane of the general formula (I)
AₓSi(N R¹R²)₄₋ₓ (I)
wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue furthermore, A may be an aromatic residue, preferably A is independently selected from optionally substituted C₁-C₂₀-alkyl-, C₆-C₂₄-aryl, in particular preferred a methyl, ethyl, propyl, vinyl, allyl, butyl or phenyl residue,
X is an integer of 0, 1 or 2
R¹ and R² are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R¹ and R² may be an aromatic residue, preferably R₁ and R₂ is independently selected from optionally substituted C₁-C₂₀-alkyl-, C₆-C₂₄-aryl, in particular preferred a methyl, ethyl, propyl, butyl, hexyl or phenyl residue.

12. The method according to any one of claims 9 to 10 wherein the Si-N precursor having at least two amino groups is an oligo- or polysilazane.

13. The method according to any one of claims 9 to 12 wherein the sensitive material, in particular, the light-, pH- and/or temperature-sensitive material is a photosensitve material, biological material, like a photosynthetically active material.

14. The silica-based gel with entrapped sensitive material according to any one of claims 1 to 9 for use in biosensors, photonic devices, medical, pharmaceutical, agricultural, food and cosmetic applications, optical information processing bioreactors, photovoltaics and optical materials.
